# EUROPEAN PATENT SPECIFICATION

(11) **EP 3 946 038 B1**
(45) Date of publication and mention of the grant of the patent: **27.07.2022**
(21) Application number: 20713274.7
(22) Date of filing: 20.03.2020
(51) Int. Cl.: A61B 5/389, A61B 5/11, A61B 5/00

(54) **ASSESSING MUSCLE FATIGUE**
BEURTEILUNG VON MUSKELERMÜDUNG
ÉVALUATION DE LA FATIGUE MUSCULAIRE

(30) Priority: 27.03.2019 EP 19165404
(43) Date of publication of application: 09.02.2022
(73) Proprietor: Koninklijke Philips N.V., 5656 AG Eindhoven (NL)
(72) Inventor: SARTOR, Francesco, 5656 AE Eindhoven (NL)
(74) Representative: Philips Intellectual Property & Standards
(86) International application number: PCT/EP2020/057738
(87) International publication number: WO 2020/193393

(56) References cited:
- WO-A1-2013/039391
- MORITANI T ET AL: "Electromechanical changes during electrically induced and maximal voluntary contractions: Surface and intramuscular EMG responses during sustained maximal voluntary contraction", EXPERIMENTAL NEUROLOGY, ELSEVIER, AMSTERDAM, NL, vol. 88, no. 3, 1 June 1985 (1985-06-01), pages 484-499, XP026226395, ISSN: 0014-4886, DOI: 10.1016/0014-4886(85)90065-2 [retrieved on 1985-06-01]
- JONSSON B: "Measurement and evaluation of local muscular strain in the shoulder during constrained work.", JOURNAL OF HUMAN ERGOLOGY SEP 1982, vol. 11, no. 1, September 1982 (1982-09), pages 73-88, XP002794201, ISSN: 0300-8134

## Description

### FIELD OF THE INVENTION

The invention relates to the assessment of muscle fatigue in at least one muscle of a subject, and in particular to a computer-implemented method, apparatus and computer program product for assessing muscle fatigue.

### BACKGROUND OF THE INVENTION

Muscle fatigue and muscle fatigability is useful information in several domains, such as sports, rehabilitation from injury or surgery, patient care and deterioration, independent living (particularly for elderly people), and in the context of occupational physiology.

Conventionally, muscle fatigue can be inferred by the use of surface electromyography (sEMG) and fatiguing protocols (repeated contractions of the muscle) and/or electrical-stimulation of the muscle in the laboratory under controlled conditions and using specialised equipment.

The paper "Detection of muscle fatigue using wearable (MYO) surface electromyography based control device" by T. M. Nourhan, M. Piechnick, J. Falkenberg and T. Nazmy, 8th International Conference on Information Technology (ICIT), Amman, 2017, pp. 44-49 describes the assessment of fatigue using sEMG implemented in a wearable device outside the laboratory. The paper "Electromechanical changes during electrically induced and maximal voluntary contractions: Surface and intramuscular EMG responses during sustained maximal voluntary contraction" by T. Mortani ET AL, EXPERIMENTAL NEUROLOGY, ELSEVIER, Amsterdam, NL, vol. 88, no. 3, 1 June 1985 (1985-06-01), pages 484-499, ISSN: 0014-4886, DOI: 10.1016/0014-4886(85)90065-2 describes another assessment technique of muscle fatigue.

However, the conventional devices are limited by requiring some contextual information about the muscle contraction. For example, whether the contraction was in response to a light, medium or heavy load. Whether the contraction was isometric or dynamic. Whether the contraction was passive (eccentric) or active (concentric). Moreover, sEMG-derived features such as root mean square (RMS) and integrated electromyography (iEMG) can provide misleading information as they are not robust against movement artefacts and signal noise, and they need to be normalised.

Therefore there is a need for improvements in the assessment of muscle fatigue.

### SUMMARY OF THE INVENTION

In particular there is a need for improvements in the assessment of muscle fatigue that can enable muscle fatigue to be assessed over time outside of a laboratory or clinical environment (e.g. during activities of daily living, ADL).

In addition or alternatively, there is a need for improvements in the assessment of muscle fatigue that can enable muscle fatigue to be assessed using sensors other than sEMG and/or features that have to be derived from sEMG measurements.

In addition or alternatively, there is a need for improvements in the assessment of muscle fatigue that can enable muscle fatigue to be assessed without requiring a calibration by the subject and/or without requiring contextual information about any observed muscle contractions.

Therefore, according to a first specific aspect, there is provided a computer-implemented method for assessing muscle fatigue in at least one muscle of a subject. The method comprises (i) obtaining a first set of measurements of muscle contractions of the at least one muscle for a first time period; (ii) forming a first frequency distribution from values of a muscle contraction feature determined from the first set of measurements; (iii) determining a first distribution fit of the first frequency distribution; (iv) determining whether the first distribution fit is statistically stable; (v) if the first distribution fit is determined to be statistically stable, determining, from the first distribution fit, a first value for a distribution fit feature; (vi) comparing the first value to a second value for the distribution fit feature to determine a measure of the fatigue of the at least one muscle during the first time period, wherein the second value for the distribution fit feature relates to a second distribution fit of a second frequency distribution, wherein the second frequency distribution is formed from values of the muscle contraction feature determined from a second set of measurements of muscle contractions of the at least one muscle for a second time period that is different to the first time period; and (vii) outputting a signal representing the determined measure of the fatigue. Therefore the first aspect provides that muscle fatigue can be assessed outside of a laboratory or clinical environment using any sensor that can measure muscle contractions, without requiring a calibration by the subject and without requiring contextual information about any observed muscle contractions.

In some embodiments the measurements of muscle contractions are obtained by one or more of: a surface electromyography (sEMG) sensor, a mechanomyography (MMG) sensor, an accelerometer, a strain gauge sensor, a piezoelectric sensor, a stretch sensor or a deformation sensor.

In some embodiments the first set of measurements comprises measurements of at least a minimum number of muscle contractions of the at least one muscle.

In some embodiments the method further comprises determining the minimum number of muscle contractions required based on a number of muscle contractions of the at least one muscle during the second time period.

In some embodiments the first frequency distribution is formed by processing the first set of measurements to determine values of the muscle contraction feature for muscle contractions in the first time period; and forming the first frequency distribution from the determined values of the muscle contraction feature. The muscle contraction feature can be any of intensity of the muscle contraction, duration of the muscle contraction, and tremors in the muscle contraction.

In some embodiments step (iv) comprises: determining a measure of the variability of the first distribution fit; and comparing the determined measure of the variability to a threshold value. The first distribution fit can be determined to be statistically stable if the determined measure of the variability is below the threshold value.

In some embodiments the distribution fit feature comprises any of: a maximum value of the muscle contraction feature; a minimum value of the muscle contraction feature; a scale of values for the first distribution fit; a measure of dispersion of the first distribution fit; a measure of the shape of the first distribution fit; a number of muscle contractions in a predetermined part of the first distribution fit; and a number of muscle contractions in the first distribution fit. In these embodiments the measure of the fatigue of the at least one muscle during the first time period can be determined based on a difference between, or ratio of, the first value of the distribution fit feature and the second value of the distribution fit feature. In alternative embodiments, the measure of the fatigue of the at least one muscle during the first time period can be determined based on a difference between, or ratio of, the maximum value of the muscle contraction feature for the first distribution fit and the maximum value of the muscle contraction feature for the second distribution fit.

In some embodiments the method further comprises, if the first distribution fit is not determined to be statistically stable, obtaining further measurements of muscle contractions of the at least one muscle; updating the first frequency distribution to include the further measurements; repeating steps (iii) and (iv) for the updated first frequency distribution.

According to a second aspect, there is provided a computer program product comprising a computer readable medium having computer readable code embodied therein, the computer readable code being configured such that, on execution by a suitable computer or processor, the computer or processor is caused to perform the method according to the first aspect or any embodiment thereof.

According to a third specific aspect, there is provided an apparatus for assessing muscle fatigue in at least one muscle of a subject. The apparatus comprises a processing unit configured to: obtain a first set of measurements of muscle contractions of the at least one muscle for a first time period; form a first frequency distribution from values of a muscle contraction feature determined from the first set of measurements; determine a first distribution fit of the first frequency distribution; determine whether the first distribution fit is statistically stable; determine, from the first distribution fit, a first value for a distribution fit feature if the first distribution fit is determined to be statistically stable; compare the first value to a second value for the distribution fit feature to determine a measure of the fatigue of the at least one muscle during the first time period, wherein the second value for the distribution fit feature relates to a second distribution fit of a second frequency distribution, wherein the second frequency distribution is formed from values of the muscle contraction feature determined from a second set of measurements of muscle contractions of the at least one muscle for a second time period that is different to the first time period; and output a signal representing the determined measure of the fatigue. Therefore the third aspect provides that muscle fatigue can be assessed outside of a laboratory or clinical environment using any sensor that can measure muscle contractions, without requiring a calibration by the subject and without requiring contextual information about any observed muscle contractions.

In some embodiments the measurements of muscle contractions are obtained from one or more of: a surface electromyography (sEMG) sensor, a mechanomyography (MMG) sensor, an accelerometer, a strain gauge sensor, a piezoelectric sensor, a stretch sensor or a deformation sensor.

In some embodiments the first set of measurements comprises measurements of at least a minimum number of muscle contractions of the at least one muscle.

In some embodiments the processing unit is further configured to determine the minimum number of muscle contractions required based on a number of muscle contractions of the at least one muscle during the second time period.

In some embodiments the processing unit is configured to form the first frequency distribution by processing the first set of measurements to determine values of the muscle contraction feature for muscle contractions in the first time period; and forming the first frequency distribution from the determined values of the muscle contraction feature. The muscle contraction feature can be any of intensity of the muscle contraction, duration of the muscle contraction, and tremors in the muscle contraction.

In some embodiments the processing unit is further configured to determine whether the first distribution fit is statistically stable by: determining a measure of the variability of the first distribution fit and comparing the determined measure of the variability to a threshold value. The first distribution fit can be determined to be statistically stable if the determined measure of the variability is below the threshold value.

In some embodiments the distribution fit feature comprises any of: a maximum value of the muscle contraction feature; a minimum value of the muscle contraction feature; a scale of values for the first distribution fit; a measure of dispersion of the first distribution fit; a measure of the shape of the first distribution fit; a number of muscle contractions in a predetermined part of the first distribution fit; and a number of muscle contractions in the first distribution fit. In these embodiments the measure of the fatigue of the at least one muscle during the first time period can be determined based on a difference between, or ratio of, the first value of the distribution fit feature and the second value of the distribution fit feature. In alternative embodiments, the measure of the fatigue of the at least one muscle during the first time period can be determined based on a difference between, or ratio of, the maximum value of the muscle contraction feature for the first distribution fit and the maximum value of the muscle contraction feature for the second distribution fit.

In some embodiments the processing unit is further configured to: obtain further measurements of muscle contractions of the at least one muscle if the first distribution fit is not determined to be statistically stable; update the first frequency distribution to include the further measurements; determine a first distribution fit of the updated first frequency distribution; and determine whether the updated first distribution fit is statistically stable.

According to a fourth aspect, there is provided a system that comprises: a device that is to be carried or worn by a subject and that comprises a muscle contraction sensor for measuring contractions of one or more muscles of the subject; and an apparatus according to the third aspect or any embodiment thereof.

In some embodiments the apparatus is part of the device. In alternative embodiments, the apparatus is separate from the device.

These and other aspects will be apparent from and elucidated with reference to the embodiment(s) described hereinafter.

### BRIEF DESCRIPTION OF THE DRAWINGS

Exemplary embodiments will now be described, by way of example only, with reference to the following drawings, in which:
Fig. 1 is a block diagram illustrating an apparatus according to an exemplary embodiment;
Fig. 2 is a flow chart illustrating a method according to an exemplary embodiment;
Fig. 3 is an illustration of an exemplary frequency distribution formed from a set of muscle contraction measurements;
Fig. 4 is an illustration of an exemplary distribution fit of the frequency distribution shown in Fig. 3;
Fig. 5 is an illustration of the frequency distribution of Fig. 4 and another exemplary frequency distribution and corresponding distribution fit; and
Fig. 6 shows the frequency distributions and distribution fits of Fig. 4 with respective values for a distribution fit feature.

### DETAILED DESCRIPTION OF EMBODIMENTS

According to the techniques described herein, improvements in the assessment of muscle fatigue are provided that can any of: enable muscle fatigue to be assessed over time outside of a laboratory or clinical environment, enable muscle fatigue to be assessed using sensors other than surface electromyography (sEMG) and/or features that have to be derived from sEMG measurements, enable muscle fatigue to be assessed without requiring a calibration by the subject and/or without requiring contextual information about any observed muscle contractions. The subject to which the techniques described herein are applied can be a person or animal. In some embodiments the muscle(s) for which fatigue is determined can be any of the muscles in the forearm (e.g. flexor pollicis longus, flexor digitorum profundus, extensor carpi radialis longus and brevis, extensor digitorum, extensor carpi ulnaris), or any muscle in the leg or back.

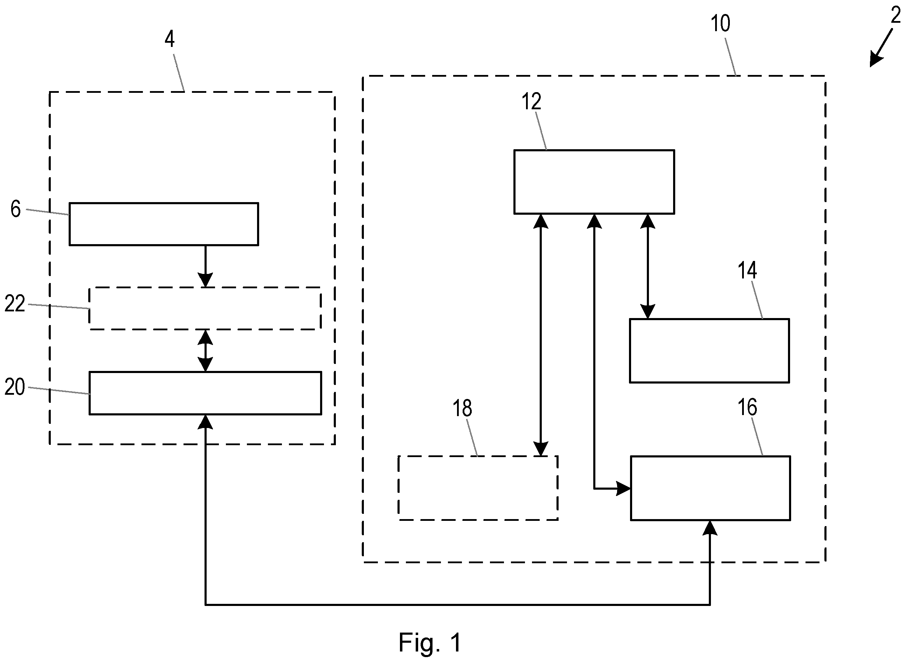

Fig. 1 illustrates a system 2 according to an exemplary embodiment of the teachings presented herein. In this embodiment the system 2 comprises a device 4 that is carried or worn by the subject and that includes a muscle contraction sensor 6 for measuring contractions of one or more muscles of a subject. The system 2 also comprises an apparatus 10 that receives the measurements of muscle contractions and analyses the measurements to determine a measure of the fatigue of the one or more muscles.

The device 4 can be in any form suitable to enable the subject to carry or wear the device 4. For example, the device 4 may be in the form of a watch or smartwatch, a smartphone, a bracelet, a pendant, a necklace, a chest band, an arm band, a leg band, integrated into an item of clothing, etc. Typically, the device 4 is to be carried or worn in proximity to or on the part of the body of the subject in which a muscle or muscles to be assessed are located (e.g. the device 4 could be worn on a leg to measure fatigue in a leg muscle or leg muscles). As the device 4 is portable, it enables measurements of muscle contractions to be obtained while the subject is performing their daily activities (e.g. walking, climbing stairs, etc.).

In some embodiments, as shown in Fig. 1, the apparatus 10 can be separate from the device 4. In these embodiments, the apparatus 10 can be any type of electronic device or computing device that can communicate with, or otherwise receive the muscle contraction measurements directly or indirectly from, the device 4. For example the apparatus 10 can be, or be part of, a computer, a laptop, a tablet, a smartphone, a smartwatch, etc., and as such may be an apparatus that is present or used in the home or care environment of the subject. In other implementations, the apparatus 10 can be an apparatus that is remote from the subject, and remote from the home or care environment of the subject. For example, the apparatus 10 can be a server, for example a server in a data centre (also referred to as being 'in the cloud'). In alternative embodiments, the apparatus 10 (and in particular the functionality of the apparatus 10 as described herein) can be integral with the device 4. Therefore the apparatus 10 can also be carried or worn by the subject as part of the device 4.

The muscle contraction sensor 6 can include any type of sensor(s) for measuring the contractions of one or more muscles of a subject, or for providing measurements representative of the muscle contraction of a subject. The muscle contraction sensor 6 generates and outputs a muscle contraction signal representing measurements of muscle contractions of the subject over time (e.g. over a first time period). The muscle contraction signal can comprise a time series of muscle contraction measurements (a time series of samples) covering the time period. The muscle contraction sensor 6 can use any desired sampling frequency, for example 50 measurements per second (50 Hz), 60 Hz or 100 Hz. The muscle contraction sensor 6 can be a sensor that measures an electrical component associated with muscle contractions (e.g. an sEMG sensor), a sensor that measures a mechanical component associated with muscle contractions (e.g. a mechanomyography (MMG) sensor, a sensor that measures an amount or level of movement associated with muscle contractions (e.g. an accelerometer), or a sensor that responds to geometrical changes caused by muscle contractions (e.g. a strain gauge, a piezoelectric sensor, a sensor that includes conductive rubber, or any other type of stretch or deformation sensor).

The apparatus 10 includes a processing unit 12 that controls the operation of the apparatus 10 and that can be configured to execute or perform the methods described herein. In particular, the processing unit 12 can obtain the muscle contraction signal/muscle contraction measurements and process them to assess the fatigue of the muscle(s). The processing unit 12 can be implemented in numerous ways, with software and/or hardware, to perform the various functions described herein. The processing unit 12 may comprise one or more microprocessors or digital signal processor (DSPs) that may be programmed using software or computer program code to perform the required functions and/or to control components of the processing unit 12 to effect the required functions. The processing unit 12 may be implemented as a combination of dedicated hardware to perform some functions (e.g. amplifiers, pre-amplifiers, analog-to-digital convertors (ADCs) and/or digital-to-analog convertors (DACs)) and a processor (e.g., one or more programmed microprocessors, controllers, DSPs and associated circuitry) to perform other functions. Examples of components that may be employed in various embodiments of the present disclosure include, but are not limited to, conventional microprocessors, DSPs, application specific integrated circuits (ASICs), and field-programmable gate arrays (FPGAs).

The processing unit 12 is connected to a memory unit 14 that can store data, information and/or signals (including muscle contraction measurements) for use by the processing unit 12 in controlling the operation of the apparatus 10 and/or in executing or performing the methods described herein. In some implementations the memory unit 14 stores computer-readable code that can be executed by the processing unit 12 so that the processing unit 12 performs one or more functions, including the methods described herein. In particular embodiments, the program code can be in the form of an application for a smartwatch, a smartphone, tablet, laptop or computer. The memory unit 14 can comprise any type of non-transitory machine-readable medium, such as cache or system memory including volatile and non-volatile computer memory such as random access memory (RAM) static RAM (SRAM), dynamic RAM (DRAM), read-only memory (ROM), programmable ROM (PROM), erasable PROM (EPROM) and electrically erasable PROM (EEPROM), implemented in the form of a memory chip, an optical disk (such as a compact disc (CD), a digital versatile disc (DVD) or a Blu-Ray disc), a hard disk, a tape storage solution, or a solid state device, including a memory stick, a solid state drive (SSD), a memory card, etc.

In the embodiment of the system 2 shown in Fig. 1, as the apparatus 10 is separate from the device 4 that includes the muscle contraction sensor 6, the apparatus 10 also includes interface circuitry 16 for enabling a data connection to and/or data exchange with other devices, including device 4, and optionally any one or more of servers, databases, user devices, and sensors. The connection may be direct or indirect (e.g. via the Internet), and thus the interface circuitry 16 can enable a connection between the apparatus 10 and a network, such as the Internet, or between the apparatus 10 and device 4, via any desirable wired or wireless communication protocol. For example, the interface circuitry 16 can operate using WiFi, Bluetooth, Zigbee, or any cellular communication protocol (including but not limited to Global System for Mobile Communications (GSM), Universal Mobile Telecommunications System (UMTS), Long Term Evolution (LTE), LTE-Advanced, etc.). In the case of a wireless connection, the interface circuitry 16 (and thus apparatus 10) may include one or more suitable antennas for transmitting/receiving over a transmission medium (e.g. the air). Alternatively, in the case of a wireless connection, the interface circuitry 16 may include means (e.g. a connector or plug) to enable the interface circuitry 16 to be connected to one or more suitable antennas external to the apparatus 10 for transmitting/receiving over a transmission medium (e.g. the air). The interface circuitry 16 is connected to the processing unit 12 to enable information or data received by the interface circuitry 16 to be provided to the processing unit 12, and/or information or data from the processing unit 12 to be transmitted by the interface circuitry 16.

The interface circuitry 16 can be used to receive muscle contraction measurements generated by the muscle contraction sensor 6.

In some embodiments, the interface circuitry 16 can be used to output a result of the processing by the processing unit 12, for example an indication of the muscle fatigue, and/or information relating to the determined muscle fatigue.

In some embodiments, the apparatus 10 comprises a user interface 18 that includes one or more components that enables a user of apparatus 10 (e.g. the subject, or a care provider for the subject) to input information, data and/or commands into the apparatus 10 (e.g. for starting or enabling the analysis of muscle contraction measurements according to the techniques described herein), and/or enables the apparatus 10 to output information or data to the user of the apparatus 10. An output may be an audible, visible and/or tactile indication of the level of muscle fatigue, for example. The user interface 18 can comprise any suitable input component(s), including but not limited to a keyboard, keypad, one or more buttons, switches or dials, a mouse, a track pad, a touchscreen, a stylus, a camera, a microphone, etc., and the user interface 18 can comprise any suitable output component(s), including but not limited to a display screen, one or more lights or light elements, one or more loudspeakers, a vibrating element, etc.

It will be appreciated that a practical implementation of apparatus 10 may include additional components to those shown in Fig. 1. For example the apparatus 10 may also include a power supply, such as a battery, or components for enabling the apparatus 10 to be connected to a mains power supply.

As noted above, the muscle contraction sensor 6 is part of device 4, which is separate from the apparatus 10 in the embodiment shown in Fig. 1. In order for the muscle contraction measurements to be communicated from the device 4 to the apparatus 10, the device 4 comprises interface circuitry 20. The interface circuitry 20 may be implemented in a similar way to the interface circuitry 16 in the apparatus 10.

In some embodiments, the device 4 can also include a processing unit 22 for controlling the operation of the device 4. This processing unit 22 can also be used to perform some pre-processing of the muscle contraction measurements before they are communicated to the apparatus 10, for example the measurements can be filtered to reduce or remove a noise component or artefacts. The processing unit 22 may be implemented in a similar way to the processing unit 12 in the apparatus 10.

It will be appreciated that a practical implementation of device 4 may include additional components to those shown in Fig. 1. For example the device 4 may also include a power supply, preferably a battery so that the device 4 is portable, or components for enabling the device 4 to be connected to a mains power supply.

In alternative embodiments of the system 2 where the apparatus 10 is part of the device 4, it will be appreciated that only one processing unit 12/22 may be present, and interface circuitry is not required to communicate the muscle contraction measurements to the processing unit 12.

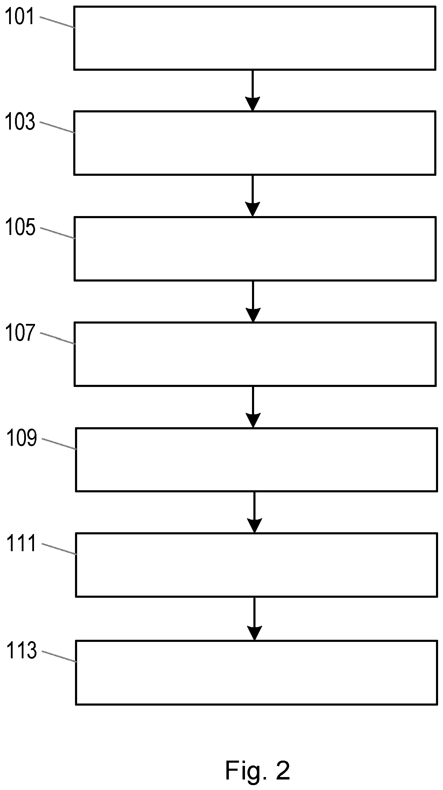

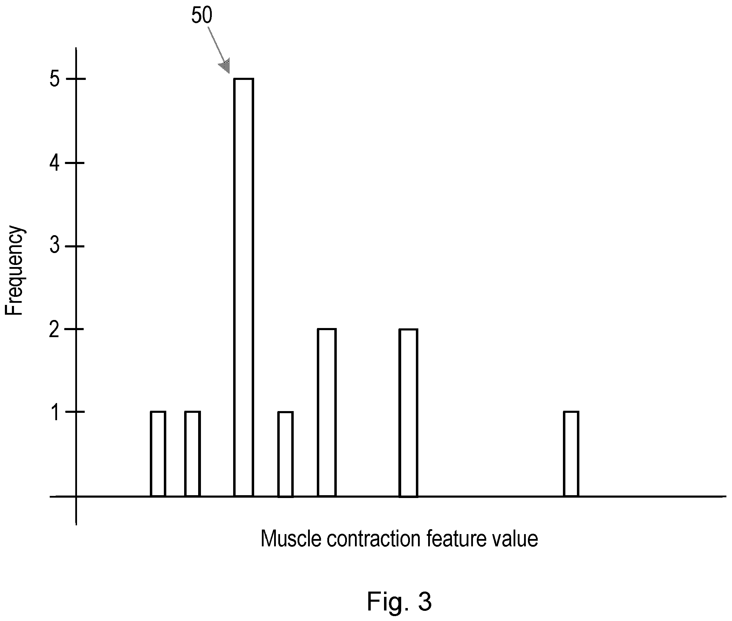

The flow chart in Fig. 2 illustrates an exemplary method according to the techniques described herein. One or more of the steps of the method can be performed by the processing unit 12 in the apparatus 10, in conjunction with any of the memory unit 14, interface circuitry 16 and user interface 18 as appropriate. The processing unit 12 may perform the one or more steps in response to executing computer program code, that can be stored on a computer readable medium, such as, for example, the memory unit 14. The flow chart in Fig. 2 is described with reference to the Figs. 3-6.

In a first step, step 101, the processing unit 12 obtains a first set of measurements of muscle contractions of at least one muscle of the subject during at least a first time period. In some embodiments the at least one muscle any of the muscles in the forearm (e.g. flexor pollicis longus, flexor digitorum profundus, extensor carpi radialis longus and brevis, extensor digitorum, extensor carpi ulnaris), or any muscle in the leg or back. The measurements of muscle contractions are obtained from a muscle contraction signal that is output by the muscle contraction sensor 6 in device 4. The device 4 is being carried or worn by the subject at least during the first time period. The processing unit 12 can obtain the muscle contraction signal directly from the muscle contraction sensor 6 or indirectly from the muscle contraction sensor 6 (e.g. via interface circuitry 16 and interface circuitry 20). In these embodiments the processing unit 12 may be able to process the measurements as they are received (e.g. in real-time or near-real-time) to assess muscle fatigue. Alternatively, the processing unit 12 can retrieve the muscle contraction signal from the memory unit 14. In some embodiments the processing unit 12 can receive the muscle contraction signal representing the muscle contractions of the at least one muscle during the first time period after the first time period has passed. Alternatively, the processing unit 12 can receive the muscle contraction signal over the course of the first time period as the muscle contractions are measured.

In some embodiments the muscle contraction measurements includes measurements of at least a minimum number of muscle contractions (for example at least 10 contractions). The first time period therefore preferably has a duration that is long enough to cover at least the minimum number of muscle contractions. The duration of the first time period can be, for example, 1 minute (e.g. if the subject is engaged in an intense physical activity), at least 10 minutes, 1 hour, 4 hours, a morning, an afternoon, an evening, a day, etc. In some embodiments, the processing unit 12 can continue obtaining a set of measurements of muscle contractions via the muscle contraction sensor 6 until at least the minimum number of muscle contractions have been measured.

In some embodiments the minimum number of muscle contractions required may be determined on a subject-by-subject basis. The minimum number of muscle contractions may be determined based on a typical or average number of muscle contractions for the subject per time period (e.g. per hour, per day, etc.).

In step 103, the processing unit 12 forms a frequency distribution (referred to herein as a "first frequency distribution") from values of a muscle contraction feature determined from the first set of measurements. As is known, a frequency distribution is a function, such as a histogram or plot, that indicates the frequency (i.e. number of times) that a value or range of values of a variable occurs in a dataset. In the present case, the frequency distribution indicates the number of times a value or range of values of the muscle contraction feature occurs in the first set of measurements.

Thus, step 103 can comprise processing the first set of measurements to determine values of the muscle contraction feature for each muscle contraction that occurred in the first time period and that can be identified in the muscle contraction signal. The first frequency distribution is then formed from these values.

In some embodiments the muscle contraction feature is any of the intensity of a muscle contraction, the duration of a muscle contraction, and tremors in the muscle contraction. In other embodiments the muscle contraction feature is muscle contraction steady state, rising slope or decay. In preferred embodiments, the muscle contraction feature is the intensity of a muscle contraction, and this can be determined from the power of the muscle contraction signal, e.g. the root mean square (RMS). Where the muscle contraction signal is obtained by an sEMB sensor, the intensity of a muscle contraction can be determined by calculating the root mean square (RMS) of the part of the sEMB signal (or the part of the first set of measurements) relating to that muscle contraction. Where the muscle contraction signal is obtained by an accelerometer, the intensity of a muscle contraction can be determined by calculating the root mean square (RMS) of the part of the acceleration signal (or the part of the first set of measurements) relating to that muscle contraction. The duration of a muscle contraction can be determined by identifying the onset and end of a muscle contraction in the muscle contraction signal. As an example, the onset and end of a muscle contraction can be identified from the RMS of the muscle contraction signal, and in particular where the RMS rises above a threshold (the onset) and falls below the threshold (the end). This threshold can be, for example, twice the standard deviation of the RMS of the muscle contraction signal. A muscle tremor can be identified from the muscle contraction signal using a frequency domain analysis (e.g. a fast Fourier transform, FFT), with the tremors corresponding to the high(er) frequency components of the signal. Muscle contraction steady-state occurs when the muscle contraction is sustained within a certain coefficient of variation. This could be assessed in the muscle contraction signal using a moving mean and moving standard deviation function. The rising slope, e.g. in the case of an EMG signal, is the part of the signal where the EMG signal goes from a rest value to a muscle contraction value. The rising slope can be identified by analysing a first order derivative of the EMG signal. The decay is the opposite side of the muscle contraction peak to the rising slope, and can also be identified by analysing a first order derivative of the EMG signal.

Step 103 can be performed once measurements for the minimum number of muscle contractions have been obtained, or step 103 can be performed as the muscle contractions occur and the frequency distribution gradually built up from the measurements over time.

Fig. 3 is an illustration of an exemplary frequency distribution 50 in the form of a histogram formed from a first set of measurements of muscle contractions. In particular, the histogram 50 in Fig. 3 is a frequency distribution for 13 values of the intensity of muscle contractions occurring in the first time period.

Next, in step 105, the processing unit 12 determines a distribution fit of the first frequency distribution. This distribution fit is referred to as the "first distribution fit" herein. The first distribution fit is a probability density function that is fitted to the data in the first frequency distribution.

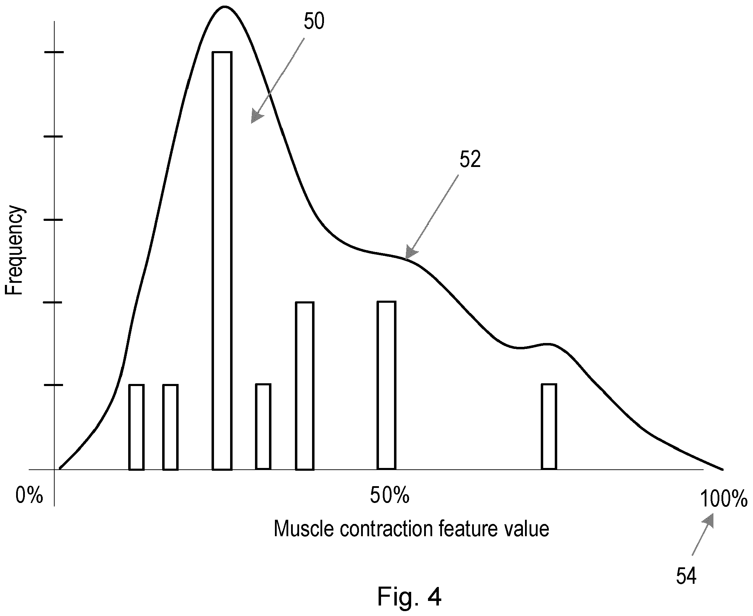

Fig. 4 is an illustration of an exemplary distribution fit 52 for the histogram 50 shown in Fig. 3.

Next, in step 107, the processing unit 12 determines whether the first distribution fit is statistically stable. That is, in step 107 the processing unit 12 determines if the first frequency distribution is formed from enough data (i.e. enough values of the muscle contraction feature) for the first frequency distribution to be 'coherent' or 'stable', so the addition of further data to the frequency distribution would not substantially change the shape of the first frequency distribution or the first distribution fit.

In some embodiments of step 107, the processing unit 12 determines a measure of the variability of the first distribution fit. The measure of variability is then compared to a threshold value to determine if the first distribution fit is statistically stable. In some embodiments, the first distribution fit is determined to be statistically stable if the measure of the variability is below the threshold value, and the first distribution fit is determined to be statistically unstable if the measure of the variability is above the threshold value. The measure of variability can be a variability of the first frequency distribution, a variance of the first frequency distribution, a coefficient of variation of the first frequency distribution, or standard deviation of the first frequency distribution. Those skilled in the art will be aware of various types of variability measures that can be used to determine if a distribution fit is statistically stable.

The histogram 50 in Fig. 4 includes 13 values of the intensity of muscle contractions occurring in the first time period, and this is sufficient for the first distribution fit 52 in Fig. 4 to be deemed stable.

If it is determined in step 107 that the first distribution fit is not statistically stable, then values of the muscle contraction feature for further muscle contractions are obtained (e.g. by measuring further muscle contractions or by evaluating further muscle contractions in already-obtained measurements) and added to the first frequency distribution. That is, further measurements of muscle contractions of the at least one muscle are obtained (e.g. measurements of further muscle contractions in the first time period, muscle contractions in a time period following the first time period, or muscle contractions occurring on an ongoing basis after the end of the first time period), and the first frequency distribution is updated to include the further values of the muscle contraction feature determined from the further measurements. An updated distribution fit is then determined for the updated first frequency distribution according to step 105 and the updated distribution fit is then checked for stability according to step 107. If stable, the updated distribution fit is analysed according to step 109 below. If the updated distribution fit is still not statistically stable, this process repeats until stability is achieved.

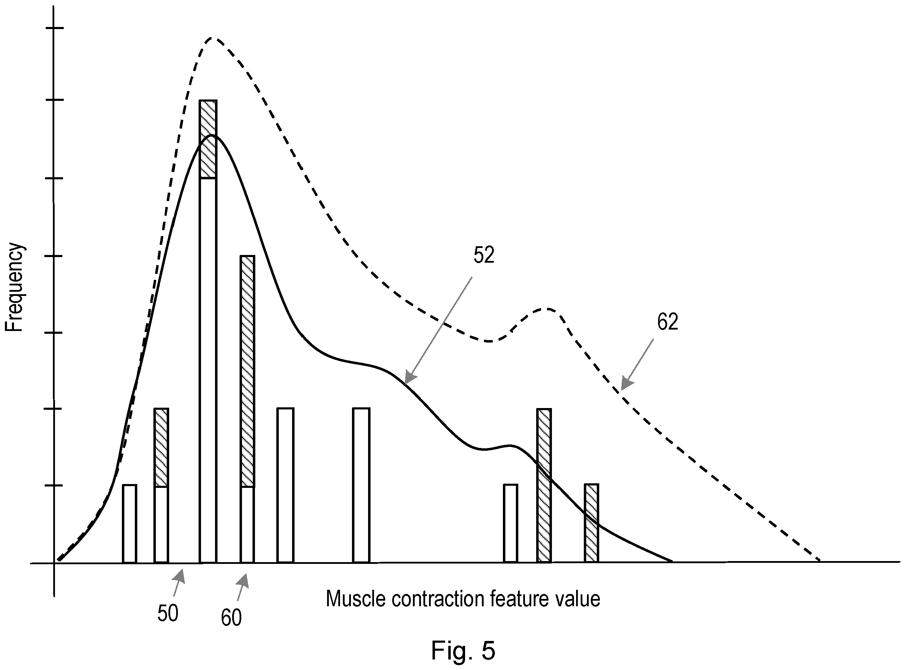

For ease of illustration, and notwithstanding that the distribution fit in Fig. 4 is itself statistically stable, Fig. 5 shows the effect of updating a histogram with further values of the muscle contraction feature and the effect that this has on the distribution fit. Thus, Fig. 5 shows an exemplary updated histogram 60 that is statistically stable and that includes the 13 values of the intensity of muscle contractions occurring in the first time period that are present in histogram 50 (shown by the 'unfilled' columns in the histogram 60), and 8 further values of the intensity of muscle contractions in the first time period (or a subsequent time period), as shown by the 'hatched' parts of the columns in the histogram 60. Fig. 5 also shows the distribution fit 52 from Fig. 4 for the 13 original values (which this fit 52 being statistically stable as noted above), and Fig. 5 also shows a distribution fit 62 for the histogram 60 (i.e. a distribution fit for the full set of 21 values of the intensity). It can be seen that although both distribution fits 52, 62 are statistically stable, the distribution fits 52, 62 differ mainly on the right side of the histograms, where histogram 60 shows a bi-modal distribution.

If the first distribution fit (whether updated or otherwise) is determined to be statistically stable, the first distribution fit can be used as a 'template' of the muscle contractions during the first time period for comparison with a distribution fit formed from muscle contraction measurements obtained during a different time period. This comparison can provide an indication of the muscle fatigue of the at least one muscle in the first time period compared to the fatigue in the other time period.

Thus, in step 109 the processing unit 12 determines a value for a distribution fit feature from the statistically-stable first distribution fit. The value for the distribution fit feature determined from the statistically-stable first distribution fit is referred to as the "first value" herein. In some embodiments the distribution fit feature is based on the muscle contraction feature determined from the first set of measurements and used to form the first frequency distribution. In other embodiments the distribution fit feature is based on a different type of muscle contraction feature to the muscle contraction feature determined from the first set of measurements and used to form the first frequency distribution. In other embodiments, the distribution fit feature is not related to any type of muscle contraction feature, and is instead related to a characteristic of the first distribution fit.

In some embodiments the distribution fit feature is any of a maximum value of a muscle contraction feature (e.g. a maximum intensity); a minimum value of a muscle contraction feature (e.g. a minimum intensity); a scale of values of the distribution fit; a measure of dispersion of the distribution fit; a measure of the shape of the distribution fit; a number of muscle contractions in a predetermined part of the distribution fit; and a number of muscle contractions in the distribution fit. A measure of dispersion of the distribution fit can be any of a measure of the standard deviation of the distribution fit, the inter-quartile range (IQR), the range of the distribution fit, the mean absolute difference of the distribution fit, the median absolute difference of the distribution fit, average absolute deviation of the distribution fit, the average deviation of the distribution fit, or the distance standard deviation. In preferred embodiments the distribution fit feature is a scale of values on the first distribution fit, e.g. 0% corresponding to a value of the muscle contraction feature at the lowest (i.e. furthest left) end of the first distribution fit, 100% corresponding to a value of the muscle contraction feature at the highest (i.e. further right) end of the first distribution fit, and the rest of the scale (e.g. 50%, 80%, etc.) defined between these scales. A scale 54 of the values of the muscle contraction feature is shown in Fig. 4 for the first distribution fit 52.

In step 111, the processing unit 12 compares the first value of the distribution fit feature to another value for the distribution fit feature obtained from measurements of muscle contractions in another time period to determine a measure of the fatigue of the at least one muscle. The 'another value' for the distribution fit feature is referred to herein as "the second value" for the distribution fit feature, and it is determined as set out in steps 101-109 from a second set of muscle contraction measurements that relate to a different (e.g. earlier) time period to the first time period. In some embodiments the earlier time period can be an immediately preceding time period (e.g. the previous hour, the morning (when the first time period is the afternoon)), or a corresponding time period on a different day (e.g. the first time period can be the morning, and the earlier time period can be the morning on a different day). In some embodiments the different time period can be a time period that overlaps with a part of the first time period (i.e. some of the values of the muscle contraction feature can be present in both distributions/distribution fits). For example the different time period can be an earlier part of the first time period (e.g. the distribution fit feature can be determined in step 111 from distribution fit 62 in Fig. 5 and compared to a distribution fit feature determined from distribution fit 52 corresponding to the measurements used to form the histogram 50 in Fig. 4). The measure of muscle fatigue can be given by a difference between the first value and the second value. In addition or alternatively, the measure of the fatigue can be given by a ratio of the first value and the second value. In either case, the difference or ratio indicates the muscle fatigue in the first time period relative to the muscle fatigue in the earlier time period.

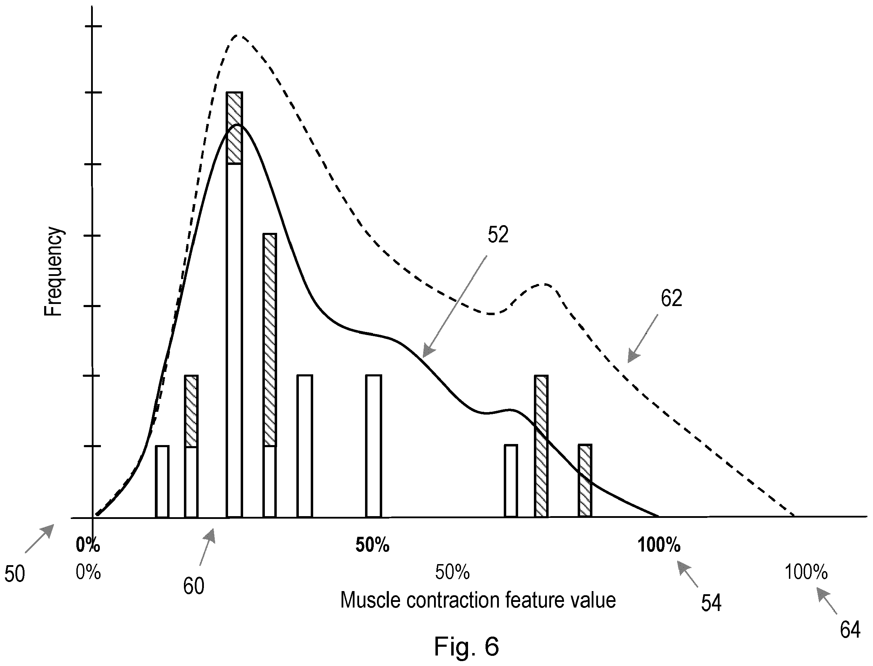

In some embodiments step 111 can comprise comparing the scale of values for the muscle contractions in the first time period to the scale of values for the muscle contractions in the earlier time period. For example the muscle contraction feature value corresponding to the 100% value (e.g. the maximum muscle contraction intensity) for the first time period can be compared to the muscle contraction feature value corresponding to the 100% value (e.g. the maximum muscle contraction intensity) for the earlier time period. If the 100% value for the first time period is higher than the 100% value for the earlier time period, then this can indicate that the at least one muscle is less fatigued in the first time period than in the earlier time period, and vice versa. Fig. 6 shows the two statistically stable frequency distributions 50 and 60 from the earlier figures, and shows the scale 54 for the distribution fit 52 (as also shown in Fig. 4), and also a scale 64 for the distribution fit 62. It can be seen in Fig. 6 that although the distribution fits 52, 62 are similar at the lower end of the distribution fits (the left side), the distribution fit 62 is wider than the distribution fit 52. This means that the scale 54 is similar to scale 64 at the lower end of the distribution fits 52, 62, but there is a more substantial difference between the distribution fits 52, 62 at the higher values of the muscle contraction feature (the right side of the histogram 60. This means that the 100% in the scale 64 is at a higher value than the 100% in the scale 54, indicating that the subject has less muscle fatigue across the muscle contractions covered by the histogram 60.

In some embodiments the comparison can comprise comparing a number of contractions in the first time period falling within a particular range of intensity to the number of contractions in the other time period falling within the particular range of intensity. Other embodiments of the comparison can be based on an absolute number of contractions. Yet other embodiments of the comparison can be based on the shape of the first distribution fit relative to the shape of the distribution fit for the other time period.

In some embodiments, step 111 can also comprise comparing the first frequency distribution (or the first distribution fit) to the frequency distribution (of distribution fit) for the other time period to determine a further measure of muscle fatigue. This comparison can use pared t Student-tests to analyse the differences between the distributions or fits, or use fitting techniques such as a method of moments, a maximum spacing estimation, a method of L-moments or a maximum likelihood method.

In some embodiments of step 111, the processing unit 12 can compare the first value of the distribution fit feature to values for the distribution fit feature obtained from measurements of muscle contractions in a plurality of other time periods to determine the measure of the fatigue of the at least one muscle during the first time period. This comparison may enable a trend in the muscle fatigue over time to be observed or derived. For example the measure of the fatigue of the muscle could be determined hour-by-hour, or day-by-day.

Finally, in step 113, the processing unit 12 outputs a signal representing the determined measure of the fatigue. The signal representing the determined measure of fatigue can be output to the user interface 18 for presentation to the subject or a user (e.g. care provider), and/or to another device or apparatus via the interface circuitry 16.

Therefore this disclosure provides improved techniques for assessing muscle fatigue. In particular the techniques enable the assessment of muscle fatigue over time outside of a laboratory or clinical environment, and particular during activities of daily living for a subject. In addition, muscle fatigue can be assessed using sensors other than sEMG, although sEMG can still be used if desired. Furthermore the techniques provide that the measure of muscle fatigue is a relative measure of muscle fatigue (i.e. relative to an earlier time period), so a calibration by the subject is not required and contextual information about any observed muscle contractions is also not required.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored or distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. A computer-implemented method for assessing muscle fatigue in at least one muscle of a subject, the method comprising:
(i) obtaining a first set of measurements of muscle contractions of the at least one muscle for a first time period;
(ii) forming a first frequency distribution from values of a muscle contraction feature determined from the first set of measurements; **characterized in that** said method further comprising
(iii) determining a first distribution fit of the first frequency distribution;
(iv) determining whether the first distribution fit is statistically stable;
(v) if the first distribution fit is determined to be statistically stable, determining, from the first distribution fit, a first value for a distribution fit feature;
(vi) comparing the first value to a second value for the distribution fit feature to determine a measure of the fatigue of the at least one muscle during the first time period, wherein the second value for the distribution fit feature relates to a second distribution fit of a second frequency distribution, wherein the second frequency distribution is formed from values of the muscle contraction feature determined from a second set of measurements of muscle contractions of the at least one muscle for a second time period that is different to the first time period; and
(vii) outputting a signal representing the determined measure of the fatigue.

2. A method as defined in claim 1, wherein the measurements of muscle contractions are obtained by one or more of: a surface electromyography (sEMG) sensor, a mechanomyography (MMG) sensor, an accelerometer, a strain gauge sensor, a piezoelectric sensor, a stretch sensor or a deformation sensor.

3. A method as defined in claim 1 or 2, wherein the first frequency distribution is formed by:
processing the first set of measurements to determine values of the muscle contraction feature for muscle contractions in the first time period; and
forming the first frequency distribution from the determined values of the muscle contraction feature.

4. A method as defined in any of claims 1-3, wherein the muscle contraction feature is any of: intensity of the muscle contraction, duration of the muscle contraction, and tremors in the muscle contraction.

5. A method as defined in any of claims 1-4, wherein step (iv) comprises:
determining a measure of the variability of the first distribution fit; and
comparing the determined measure of the variability to a threshold value;
wherein the first distribution fit is determined to be statistically stable if the determined measure of the variability is below the threshold value.

6. A method as defined in any of claims 1-5, wherein the distribution fit feature comprises any of:
a maximum value of the muscle contraction feature;
a minimum value of the muscle contraction feature;
a scale of values for the first distribution fit;
a measure of dispersion of the first distribution fit;
a measure of the shape of the first distribution fit;
a number of muscle contractions in a predetermined part of the first distribution fit; and
a number of muscle contractions in the first distribution fit.

7. A method as defined in claim 6, wherein the measure of the fatigue of the at least one muscle during the first time period is determined based on a difference between, or ratio of, the first value of the distribution fit feature and the second value of the distribution fit feature.

8. A computer program product comprising a computer readable medium having computer readable code embodied therein, the computer readable code being configured such that, on execution by a suitable computer or processor, the computer or processor is caused to perform the method of any of claims 1-7.

9. An apparatus for assessing muscle fatigue in at least one muscle of a subject, the apparatus comprising a processing unit configured to:
obtain a first set of measurements of muscle contractions of the at least one muscle for a first time period;
form a first frequency distribution from values of a muscle contraction feature determined from the first set of measurements; **characterized in that** said processing unit is further configured to
determine a first distribution fit of the first frequency distribution;
determine whether the first distribution fit is statistically stable;
determine, from the first distribution fit, a first value for a distribution fit feature if the first distribution fit is determined to be statistically stable;
compare the first value to a second value for the distribution fit feature to determine a measure of the fatigue of the at least one muscle during the first time period, wherein the second value for the distribution fit feature relates to a second distribution fit of a second frequency distribution, wherein the second frequency distribution is formed from values of the muscle contraction feature determined from a second set of measurements of muscle contractions of the at least one muscle for a second time period that is different to the first time period; and
output a signal representing the determined measure of the fatigue.

10. An apparatus as defined in claim 9, wherein the measurements of muscle contractions are obtained from one or more of: a surface electromyography (sEMG) sensor, a mechanomyography (MMG) sensor, an accelerometer, a strain gauge sensor, a piezoelectric sensor, a stretch sensor or a deformation sensor.

11. An apparatus as defined in claim 9 or 10, wherein the processing unit is configured to form the first frequency distribution by:
processing the first set of measurements to determine values of the muscle contraction feature for muscle contractions in the first time period; and
forming the first frequency distribution from the determined values of the muscle contraction feature.

12. An apparatus as defined in any of claims 9-11, wherein the muscle contraction feature is any of: intensity of the muscle contraction, duration of the muscle contraction, and tremors in the muscle contraction.

13. An apparatus as defined in any of claims 9-12, wherein the processing unit is configured to determine whether the first distribution fit is statistically stable by:
determining a measure of the variability of the first distribution fit; and
comparing the determined measure of the variability to a threshold value;
wherein the first distribution fit is determined to be statistically stable if the determined measure of the variability is below the threshold value.

14. An apparatus as defined in any of claims 9-13, wherein the distribution fit feature comprises any of:
a maximum value of the muscle contraction feature;
a minimum value of the muscle contraction feature;
a scale of values for the first distribution fit;
a measure of dispersion of the first distribution fit;
a measure of the shape of the first distribution fit;
a number of muscle contractions in a predetermined part of the first distribution fit; and
a number of muscle contractions in the first distribution fit.

15. A system, comprising:
a device that is to be carried or worn by a subject and that comprises a muscle contraction sensor for measuring contractions of one or more muscles of the subject; and
an apparatus as claimed in any of claims 9-14.

## Patentansprüche

1. Eine auf einem Computer umgesetzte Methode zum Bewerten der Muskelermüdung mindestens eines Muskels einer Person, wobei die Methode folgende Schritte umfasst:
(i) Abrufen eines ersten Satzes von Muskelkontraktionsmessungen für den mindestens einen Muskel über einen ersten Zeitraum;
(ii) Erstellen einer ersten Häufigkeitsverteilung anhand der Werte für ein Muskelkontraktionsmerkmal, das aus dem ersten Satz von Messungen ermittelt wurde; **dadurch gekennzeichnet, dass** die Methode zudem folgende Schritte umfasst:
(iii) Ermitteln einer ersten Verteilungsanpassung für die erste Häufigkeitsverteilung;
(iv) Ermitteln, ob die erste Verteilungsanpassung statistisch abgesichert ist;
(v) wenn die erste Verteilungsanpassung als statistisch abgesichert gilt, Ermitteln eines ersten Werts für ein Verteilungsanpassungsmerkmal anhand der ersten Verteilungsanpassung;
(vi) Vergleichen des ersten Werts mit einem zweiten Wert für das Verteilungsanpassungsmerkmal, um ein Maß für die Ermüdung des mindestens einen Muskels über den ersten Zeitraum zu ermitteln, wobei sich der zweite Wert für das Verteilungsanpassungsmerkmal auf eine zweite Verteilungsanpassung einer zweiten Häufigkeitsverteilung bezieht, und wobei die zweite Häufigkeitsverteilung anhand der Werte des Muskelkontraktionsmerkmals erstellt wird, die anhand eines zweiten Satzes von Muskelkontraktionsmessungen für den mindestens einen Muskel über einen zweiten Zeitraum ermittelt werden, der sich vom ersten Zeitraum unterscheidet; und
(vii) Ausgeben eines Signals, das dem ermittelten Ermüdungsgrad entspricht.

2. Eine Methode gemäß Anspruch 1, wobei die Muskelkontraktionsmessungen mit mindestens einem der folgenden Sensoren abgerufen werden: einem Oberflächen-Elektromyographie-Sensor (sEMG), einem Mechanomyographie-Sensor (MMG), einem Beschleunigungsmesser, einem Dehnungsmessser, einem piezoelektrischen Sensor, einem Ausdehnungssensor oder einem Verformungssensor.

3. Eine Methode gemäß Anspruch 1 oder 2, wobei die erste Häufigkeitsverteilung mithilfe folgender Schritte erstellt wird:
Verarbeiten des ersten Satzes von Messungen, um Werte für das Muskelkontraktionsmerkmal der Muskelkontraktionen im ersten Zeitraum zu bestimmen; und
Erstellen der ersten Häufigkeitsverteilung anhand der ermittelten Werte für das Muskelkontraktionsmerkmal.

4. Eine Methode gemäß einem der Ansprüche 1 bis 3, wobei es sich beim Muskelkontraktionsmerkmal um eines der folgenden Merkmale handelt: Intensität der Muskelkontraktion, Dauer der Muskelkontraktion und Zucken der Muskelkontraktion.

5. Eine Methode gemäß einem der Ansprüche 1 bis 4, wobei Schritt (iv) folgende Schritte umfasst:
Ermitteln des Variabilitätsmaßes der ersten Verteilungsanpassung; und
Vergleichen des ermittelten Variabilitätsmaßes mit einem Schwellenwert;
wobei die erste Verteilungsanpassung als statistisch abgesichert gilt, wenn das ermittelte Variabilitätsmaß unter dem Schwellenwert liegt.

6. Eine Methode gemäß einem der Ansprüche 1 bis 5, wobei das Verteilungsanpassungsmerkmal eines der folgenden Merkmale umfasst:
einen Maximalwert für das Muskelkontraktionsmerkmal;
einen Minimalwert für das Muskelkontraktionsmerkmal;
eine Werteskala für die erste Verteilungsanpassung;
ein Streuungsmaß für die erste Verteilungsanpassung;
ein Formmaß für die erste Verteilungsanpassung;
die Anzahl der Muskelkontraktionen für einen vorab festgelegten Teil der ersten Verteilungsanpassung; und
die Anzahl von Muskelkontraktionen in der ersten Verteilungsanpassung.

7. Eine Methode gemäß Anspruch 6, wobei das Ermüdungsmaß für den mindestens einen Muskel im ersten Zeitraum anhand der Differenz zwischen dem ersten Wert des Verteilungsanpassungsmerkmals und dem zweiten Wert des Verteilungsanpassungsmerkmals oder anhand des Verhältnisses dieser Werte ermittelt wird.

8. Ein Computerprogrammprodukt, das ein von einem Computer lesbares Medium mit von einem Computer lesbaren Code umfasst, wobei der von einem Computer lesbare Code beim Ausführen auf einem geeigneten Computer oder Prozessor diesen dazu veranlasst, die Methode gemäß einem der Ansprüche 1 bis 7 auszuführen.

9. Ein Gerät zum Bewerten der Muskelermüdung mindestens eines Muskels einer Person, wobei das Gerät eine Verarbeitungseinheit umfasst, die folgende Schritte durchführt:
Abrufen eines ersten Satzes von Muskelkontraktionsmessungen für den mindestens einen Muskel über einen ersten Zeitraum;
Erstellen einer ersten Häufigkeitsverteilung anhand der Werte für ein Muskelkontraktionsmerkmal, das aus dem ersten Satz von Messungen ermittelt wurde; **dadurch gekennzeichnet, dass** die Verarbeitungseinheit zudem folgende Schritte durchführt:
Ermitteln einer ersten Verteilungsanpassung für die erste Häufigkeitsverteilung;
Ermitteln, ob die erste Verteilungsanpassung statistisch abgesichert ist;
Anhand der ersten Verteilungsanpassung Ermitteln eines ersten Werts für ein Verteilungsanpassungsmerkmal, wenn die erste Verteilungsanpassung als statistisch abgesichert gilt;
Vergleichen des ersten Werts mit einem zweiten Wert für das Verteilungsanpassungsmerkmal, um ein Maß für die Ermüdung des mindestens einen Muskels über den ersten Zeitraum zu ermitteln, wobei sich der zweite Wert für das Verteilungsanpassungsmerkmal auf eine zweite Verteilungsanpassung einer zweiten Häufigkeitsverteilung bezieht, und wobei die zweite Häufigkeitsverteilung anhand der Werte des Muskelkontraktionsmerkmals erstellt wird, die anhand eines zweiten Satzes von Muskelkontraktionsmessungen für den mindestens einen Muskel über einen zweiten Zeitraum ermittelt werden, der sich vom ersten Zeitraum unterscheidet; und
Ausgeben eines Signals, das dem ermittelten Ermüdungsgrad entspricht.

10. Ein Gerät gemäß Anspruch 9, wobei die Muskelkontraktionsmessungen mit mindestens einem der folgenden Elemente abgerufen werden: einem Oberflächen-Elektromyographie-Sensor (sEMG), einem Mechanomyographie-Sensor (MMG), einem Beschleunigungsmesser, einem Dehnungsmessser, einem piezoelektrischen Sensor, einem Ausdehnungssensor oder einem Verformungssensor.

11. Ein Gerät gemäß Anspruch 9 oder 10, wobei die Verarbeitungseinheit die erste Häufigkeitsverteilung mithilfe folgender Schritte erstellt:
Verarbeiten des ersten Satzes von Messungen, um Werte für das Muskelkontraktionsmerkmal der Muskelkontraktionen im ersten Zeitraum zu bestimmen; und
Erstellen der ersten Häufigkeitsverteilung anhand der ermittelten Werte für das Muskelkontraktionsmerkmal.

12. Ein Gerät gemäß einem der Ansprüche 9 bis 11, wobei es sich beim Muskelkontraktionsmerkmal um eines der folgenden Merkmale handelt: Intensität der Muskelkontraktion, Dauer der Muskelkontraktion und Zucken der Muskelkontraktion.

13. Ein Gerät gemäß einem der Ansprüche 9 bis 12, wobei die Verarbeitungseinheit ermittelt, ob die erste Verteilungsanpassung statistisch abgesichert ist, indem sie folgende Schritte durchführt:
Ermitteln des Variabilitätsmaßes der ersten Verteilungsanpassung; und
Vergleichen des ermittelten Variabilitätsmaßes mit einem Schwellenwert;
wobei die erste Verteilungsanpassung als statistisch abgesichert gilt, wenn das ermittelte Variabilitätsmaß unter dem Schwellenwert liegt.

14. Ein Gerät gemäß einem der Ansprüche 9 bis 13, wobei das Verteilungsanpassungsmerkmal eines der folgenden Merkmale umfasst:
einen Maximalwert für das Muskelkontraktionsmerkmal;
einen Minimalwert für das Muskelkontraktionsmerkmal;
eine Werteskala für die erste Verteilungsanpassung;
ein Streuungsmaß für die erste Verteilungsanpassung;
ein Formmaß für die erste Verteilungsanpassung;
die Anzahl der Muskelkontraktionen für einen vorab festgelegten Teil der ersten Verteilungsanpassung; und
die Anzahl von Muskelkontraktionen in der ersten Verteilungs anpassung.

15. Ein System, das Folgendes umfasst:
ein Gerät, das von einer Person getragen wird, und das einen Muskelkontraktionssensor zum Messen der Kontraktionen mindestens eines Muskels der Person umfasst; und
ein Gerät gemäß einem der Ansprüche 9 bis 14.

## Revendications

1. Procédé mis en œuvre par ordinateur pour évaluer la fatigue musculaire dans au moins un muscle d'un sujet, le procédé consistant à :
(i) obtenir un premier ensemble de contractions musculaires d'au moins un muscle pendant une première période de temps ;
(ii) former une première distribution de fréquences à partir de valeurs d'une caractéristique de contraction musculaire déterminée à partir du premier ensemble de mesures ; **caractérisé en ce que** ledit procédé comprend en outre le fait de :
(iii) déterminer un premier ajustement de distribution de la première distribution de fréquences ;
(iv) déterminer si le premier ajustement de distribution est statistiquement stable ;
(v) si le premier ajustement de distribution est déterminé comme étant statistiquement stable, déterminer, à partir du premier ajustement de distribution, une première valeur pour une caractéristique d'ajustement de distribution ;
(vi) comparer la première valeur à une seconde valeur pour la caractéristique d'ajustement de distribution pour déterminer une mesure de la fatigue de l'au moins un muscle pendant la première période de temps, la seconde valeur pour la caractéristique d'ajustement de distribution étant relative à un second ajustement de distribution d'une seconde distribution de fréquence, la seconde distribution de fréquence étant formée à partir de valeurs de la caractéristique de contraction musculaire déterminées à partir d'un second ensemble de mesures de contractions musculaires d'au moins un muscle pendant une seconde période de temps qui est différente de la première période de temps ; et
(vii) délivrer en sortie un signal représentant la mesure déterminée de la fatigue.

2. Procédé selon la revendication 1, dans lequel les mesures de contractions musculaires sont obtenues par un ou plusieurs des éléments suivants : un capteur d'électromyographie de surface (EMGs), un capteur de mécanomyographie (MMG), un accéléromètre, un capteur à jauge de contrainte, un capteur piézoélectrique, un capteur d'étirement ou un capteur de déformation.

3. Procédé selon la revendication 1 ou 2, dans lequel la première distribution de fréquence est formée par :
le traitement du premier ensemble de mesures pour déterminer les valeurs de la caractéristique de contraction musculaire pour les contractions musculaires dans la première période de temps ; et
la formation de la première distribution de fréquence à partir des valeurs déterminées de la caractéristique de contraction musculaire.

4. Procédé selon l'une quelconque des revendications 1 à 3, dans lequel la caractéristique de contraction musculaire est l'un des éléments suivants : intensité de la contraction musculaire, durée de la contraction musculaire et tremblements dans la contraction musculaire.

5. Procédé selon l'une quelconque des revendications 1 à 4, dans lequel l'étape (iv) comprend :
la détermination d'une mesure de la variabilité du premier ajustement de distribution ; et
la comparaison de la mesure déterminée de la variabilité à une valeur seuil ;
le premier ajustement de distribution étant déterminé comme statistiquement stable si la mesure déterminée de la variabilité est en dessous de la valeur seuil.

6. Procédé selon l'une quelconque des revendications 1 à 5, dans lequel la caractéristique d'ajustement de distribution comprend l'un des éléments suivants :
une valeur maximum de la caractéristique de contraction musculaire ;
une valeur minimum de la caractéristique de contraction musculaire ;
une échelle de valeurs pour le premier ajustement de distribution ;
une mesure de dispersion du premier ajustement de distribution ;
une mesure de la forme du premier ajustement de distribution ;
un nombre de contractions musculaires dans une partie prédéterminée du premier ajustement de distribution :
et
un nombre de contractions musculaires dans le premier ajustement de distribution.

7. Procédé selon la revendication 6, dans lequel la mesure de la fatigue d'au moins un muscle durant la première période de temps est déterminée sur la base d'une différence entre, ou un rapport, de la première valeur de la caractéristique d'ajustement de distribution et de la seconde valeur de la caractéristique d'ajustement de distribution.

8. Produit de programme informatique comprenant un support lisible par ordinateur ayant un code lisible par ordinateur intégré dans celui-ci, le code lisible par ordinateur étant configuré de sorte que, lors de l'exécution par un ordinateur ou un processeur adapté, l'ordinateur ou le processeur peut exécuter le procédé de l'une quelconque des revendications 1 à 7.

9. Appareil pour évaluer la fatigue musculaire dans au moins un muscle d'un sujet, l'appareil comprenant une unité de traitement configurée pour :
obtenir un premier ensemble de contractions musculaires d'au moins un muscle pendant une première période de temps ;
former une première distribution de fréquences à partir de valeurs d'une caractéristique de contraction musculaire déterminée à partir du premier ensemble de mesures ; **caractérisé en ce que** ladite unité de traitement est en outre configurée pour déterminer un premier ajustement de distribution de la première distribution de fréquence ;
déterminer si le premier ajustement de distribution est statistiquement stable ;
déterminer, à partir du premier ajustement de distribution, une première valeur pour une caractéristique d'ajustement de distribution si le premier ajustement de distribution est déterminé comme étant statistiquement stable ;
comparer la première valeur à une seconde valeur pour la caractéristique d'ajustement de distribution pour déterminer une mesure de la fatigue d'au moins un muscle pendant la première période de temps, la seconde valeur pour la caractéristique d'ajustement de distribution étant relative à un second ajustement de distribution d'une seconde distribution de fréquence, la seconde distribution de fréquence étant formée à partir de valeurs de la caractéristique de contraction musculaire déterminées à partir d'un second ensemble de mesures de contractions musculaires d'au moins un muscle pendant une seconde période de temps qui est différente de la première période de temps ; et
délivrer en sortie un signal représentant la mesure déterminée de la fatigue.

10. Appareil selon la revendication 9, dans lequel les mesures de contractions musculaires sont obtenues à partir d'un ou de plusieurs des éléments suivants : un capteur d'électromyographie de surface (EMGs), un capteur de mécanomyographie (MMG), un accéléromètre, un capteur à jauge de contrainte, un capteur piézoélectrique, un capteur d'étirement ou un capteur de déformation.

11. Appareil selon la revendication 9 ou 10, dans lequel l'unité de traitement est configurée pour former la première distribution de fréquence par :
traitement du premier ensemble de mesures pour déterminer les valeurs de la caractéristique de contraction musculaire pour les contractions musculaires dans la première période de temps ; et
formation de la première distribution de fréquence à partir des valeurs déterminées de la caractéristique de contraction musculaire.

12. Appareil selon l'une quelconque des revendications 9 à 11, dans lequel la caractéristique de contraction musculaire est l'un des éléments suivants : intensité de la contraction musculaire, durée de la contraction musculaire et tremblements dans la contraction musculaire.

13. Appareil selon l'une quelconque des revendications 9 à 12, dans lequel l'unité de traitement est configurée pour déterminer si le premier ajustement de distribution est statistiquement stable en :
déterminant une mesure de la variabilité du premier ajustement de distribution ; et
comparant la mesure déterminée de la variabilité à une valeur seuil ;
le premier ajustement de distribution étant déterminé comme statistiquement stable si la mesure déterminée de la variabilité est en dessous de la valeur seuil.

14. Appareil selon l'une quelconque des revendications 9 à 13, dans lequel la caractéristique d'ajustement de distribution comprend l'un des éléments suivants :
une valeur maximum de la caractéristique de contraction musculaire ;
une valeur minimum de la caractéristique de contraction musculaire ;
une échelle de valeurs pour le premier ajustement de distribution ;
une mesure de dispersion du premier ajustement de distribution ;
une mesure de la forme du premier ajustement de distribution ;
un nombre de contractions musculaires dans une partie prédéterminée du premier ajustement de distribution :
et
un nombre de contractions musculaires dans le premier ajustement de distribution.

15. Procédé, comprenant :
un dispositif qui est transporté ou porté par un sujet et qui comprend un capteur de contractions musculaires pour mesurer les contractions d'un ou de plusieurs muscles du sujet ; et un appareil selon l'une quelconque des revendications 9 à 14.
